# EUROPEAN PATENT SPECIFICATION

(11) **EP 1 484 403 B1**
(45) Date of publication and mention of the grant of the patent: **27.12.2006**
(21) Application number: 03744025.2
(22) Date of filing: 07.03.2003
(51) Int. Cl.: C12N 15/29, C12N 15/09, C12N 9/02, A01H 1/00

(54) **GENE PARTICIPATING IN THE SYNTHESIS OF BRASSINOSTEROID**
AN DER SYNTHESE VON BRASSINOSTEROID BETEILIGTES GEN
GENE PARTICIPANT A LA SYNTHESE DU BRASSINOSTEROIDE

(30) Priority: 12.03.2002 JP 2002067063; 28.08.2002 JP 2002248910
(43) Date of publication of application: 08.12.2004
(73) Proprietor: Japan Science and Technology Agency, Kawaguchi-shi, Saitama 332-0012 (JP)
(72) Inventor: Tsukaya, Hirokazu, Koumuin Syukusha, Okazaki-shi, Aichi 444-0874 (JP); Kim, Gyung-Tae, Fac. Nat. Resources & Life Science, 604-714 Pusan (KR)
(74) Representative: Wilson Gunn
(86) International application number: PCT/JP2003/002755
(87) International publication number: WO 2003/076626

(56) References cited:
- KIM G T ET AL: "Changes in the shapes of leaves and flowers upon overexpression of cytochrome P450 in arabidopsis" PROCEEDINGS OF THE NATIONAL ACADEMY OF SCIENCES OF USA, NATIONAL ACADEMY OF SCIENCE, WASHINGTON, DC, US, vol. 96, 1999, pages 9433-9437, XP002967779 ISSN: 0027-8424
- KIM G-T ET AL: "The Rotundifolia3 gene of arabidopsis thaliana encodes a new member of the cytochrome P-450 family that is required for the regulated polar elongation of leaf cells" GENES AND DEVELOPMENT, COLD SPRING HARBOR LABORATORY PRESS, NEW YORK, US, vol. 12, 1998, pages 2381-2391, XP002967780 ISSN: 0890-9369
- SZEKERES M ET AL: "BRASSINOSTEROIDS RESCUE THE DEFICIENCY OF CYP90, A CYTOCHROME P450, CONTROLLING CELL ELONGATION AND DE-ETIOLATION IN ARABIDOPSIS" CELL, CELL PRESS, CAMBRIDGE, NA, US, vol. 85, 19 April 1996 (1996-04-19), pages 171-182, XP002036941 ISSN: 0092-8674
- DATABASE GENBANK [Online] 20 July 2001 SHIMADA Y. ET AL.: 'Arabidopsis thaliana mRNA for CYP90D, complete cds', XP002967778 Database accession no. (AB066286)
- KIM G. ET AL.: 'Changes in the shapes of leaves and flowers upon overexpression of cytochrome P450 in arabidopsis' PROC. NATL. ACAD. SCI. USA vol. 96, 1999, pages 9433 - 9437, XP002967779
- KIM G. ET AL.: 'The Rotundifolia3 gene of arabidopsis thaliana encodes a new member of the cytochrome P-450 family that is required for the regulated polar elongation of leaf cells' GENES & DEVELOPMENT vol. 12, 1998, pages 2381 - 2391, XP002967780

## Description

### Field of the invention:

Present invention relates to a gene participating in the synthesis of brassinosteroid, more specifically, to a novel gene (CYP90D1, SEQ ID NO: 1) controlling the final step of synthesis of brassinosteroid in combination with ROT3 gene (=CYP90C1, #51 to #1625 of SEQ ID NO: 3).

### Prior Art:

Brassinosteriods are a kind of plant hormones, which are ubiquitously distributed throughout the plant kingdom and are functional in cell elongation and cell division at extremely low concentrations, and are generic name of more than 40 kinds of analogues.

Because of their strong action on plants, brassinosteroids have been suggested to be important in their applicability to agricultural industry and many patents related to them have been disclosed (e.g., Japanese Unexamined Patent Publications, 5-222090, 6-98648, 6-340689, 8-59408, 8-81310, 8-113503, 9-97).

Researches on brassinosteroid biosynthesis have been made aggressively and the progressed elucidation of biosynthetic pathways (e.g., Fujioka et al., "Brassinosteroid biosynthesis and signal transduction" in Signal Transduction of Plant Hormones p180-189, Cell Technology Supplement, Plant Cell Technology Series 10, Shujunsha Co. Ltd, (1998)) suggests that cytochrome P450-type proteins regulate the brassinosteroid biosynthesis in plant.

The inventors already identified ROTUNDIFOLIA3 (ROT3) gene, which belongs to a family of cytochromes P450, in *Arabidopsis* (Gene & Development 12:2381-2391 (1998)), and showed that modulation of the expression of ROT3 gene resulted in morphological alterations of leaves and flowers (Proc. Natl. Acad. Sci. USA vol. 96, pp. 9433-9437 (1999)).

### Problems to be solved by the invention:

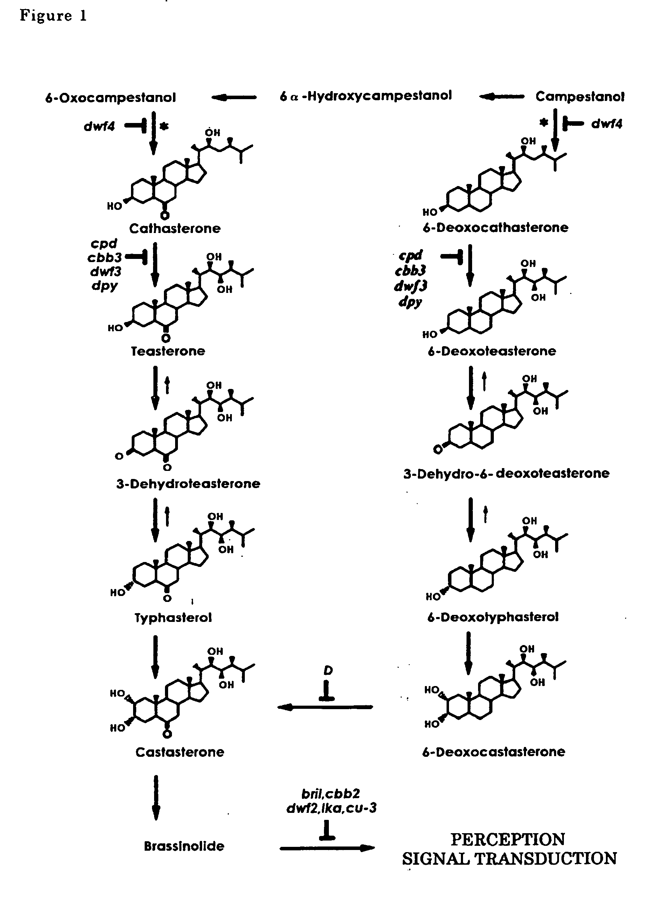

As described above, nucleic acid molecules encoding cytochrome P450-type proteins, which are involved in brassinosteroid biosynthesis, have been identified (published Japanese translation of PCT international publication for patent application (WO97/35986) No. 2000-508524). However, nucleic acid molecules previously known for the biosynthetic pathway are involved in regulation of the steps in comparatively early stage in brassinosteroid biosynthesis. Therefore, it was difficult to apply the action of the above-described nucleic acid molecules to the organ specific control or to the quantitative regulation. Furthermore, neither the enzyme proteins nor nucleic acids encoding the proteins, regulating the final step of brassinosteroid biosynthesis, have been known. The final step as used herein means the step to synthesize brasssinolide (the formula described below) from castasterone (The whole synthetic pathway of brassinosteroid is shown in Figure 1).

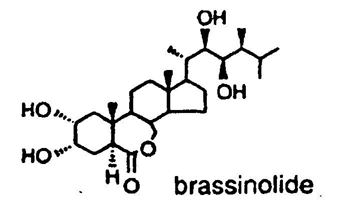

### Means to solve the problems and Detailed description of the Invention:

The inventors searched homological nucleotide sequences to ROT3, which the inventors had previously discovered, and found a nucleotide sequence that exhibits 51% identity to ROT3 gene. Examining the sequence, the inventors discovered that the sequence is a novel gene (CYP90D1, SEQ ID NO: 1), which encodes a factor regulating the final step of the brassinosteroid biosynthesis, physiologically functional in regulating the size of plant. Furthermore, the inventors discovered that the CYP90D1 gene regulates the final step of the brassinosteroid biosynthesis in combination with ROT3 (=CYP90C1) gene, then accomplished the present invention.

The present invention makes it possible to regulate the biosynthetic pathway of physiologically active brassinosteroid using ROT3 (=CYP90C1) and CYP90D1 and this possibility of regulation is the unique point that differentiates the invention from previous findings.

In other words, the expression of sole ROT3 (=CYP90C1) in a whole plant is effective only to leaves and floral organs, particularly effective in longitudinal direction. Floral organ are derived from deformed leaves, there are common pathways between them in morphological regulation by genes. On the other hand, the combination of ROT3 (=CYP90C1) and CYP90D1 is effective to a whole plant. By manipulating nucleic acid molecules of ROT3 (=CYP90C1) and CYP90D1 and proteins coded by these genes, the shape of leaves and flowers can be changed at will and at the same time, and also only the shape of flowers can be changed without changing the major part of the height of plants and the shape of leaves.

That is to say, the present invention is a polynucleotide having the nucleotide sequence of (1) or (2), and that of (3) or (4):
(1) Nucleotide sequence of SEQ ID NO: 1.
(2) Nucleotide sequences encoding either of the following proteins,
   (a) A protein having the amino acid sequence of SEQ ID NO: 2.
   (b) A protein having the amino acid of SEQ ID No: 2, wherein one amino acid is deleted, substituted or added and its expression stimulates brassinosteroid biosynthesis.
(3) Nucleotide sequence of #51 to #1625 of SEQ ID NO: 3.
(4) Nucleotide sequence encoding either of the following proteins,
   (c) A protein having the amino acid sequence of SEQ ID NO: 4.
   (d) A protein having the amino acid sequence of SEQ ID NO: 4, wherein one or some amino acids are deleted, substituted or added and its expression stimulates brassinosteroid biosynthesis.

Moreover, the present invention is a polynucleotide comprising a promoter and the polynucleotide, wherein both of the above-described nucleotide sequences are linked to said promoter in forward direction; or a polynucleotide comprising a promoter and the polynucleotide, wherein at least one of nucleotide sequence of the above-described nucleotides is linked to the above-described promoter in reverse direction.

The promoter used herein will be described later in detail and includes the cauliflower mosaic virus 35S promoter, heat shock promoter, chemical-inducible promoters and others. The linking can be operated appropriately using conventional techniques of genetic engineering.

Still furthermore, the present invention is a plasmid containing the above-described polynucleotides and is also a plant transformed by the above-described polynucleotides.

Still moreover, the present invention is a plasmid containing the above-described polynucleotide. The plasmids used herein include such binary vectors as pBI-121 plasmid, Ti plasmid and others.

Also, the plants applicable by the present invention cover whole Spermatophyte.

To transform such plants, the polynucleotide of the present invention was inserted to the above-described plasmid, which may transform the above-described plants using conventional genetic engineering methods.

In addition, the present invention is a method for altering the morphology of a plant, comprising the steps of transforming a plant by the polynucleotide and enhancing or suppressing the expression of the above-described polynucleotide. Furthermore, the present invention is a method for altering the morphology of a plant, which is transformed by any of the above-described polunucleotides, comprising the step of stimulating the responsible promoter in the transformed plant. And also, the present invention is the plant with a morphology altered by any of the above-described methods.

Also, the present invention is a mixture or a complex of protein of the following (a) or (b) and a protein of the following (c) or (d):
(a) A protein having the amino acid of SEQ ID NO: 2.
(b) A protein having the amino acid sequence of SEQ ID NO: 2, wherein one amino acid is deleted, substituted or added and its expression stimulates brassinosteroid biosynthesis.
(c) A protein having the amino acid of SEQ ID NO: 4.
(d) A protein having the amino acid sequence of SEQ ID NO: 4, wherein one or some amino acid is deleted, substituted or added and its expression stimulates brassinosteroid biosynthesis.

It is possible to manipulate nucleic acid molecules of CYP90D1 and ROT3 (=CYP90C1) and proteins coded by these using the following procedures:
(1) The procedure comprises the steps of linking a manipulable promoter with DNA molecules of CYP90D1 (SEQ ID NO: 1) and ROT3 (=CYP90C1, #51 to #1625 of SEQ ID NO: 3), transducing these into a plant by way of an appropriate conventional method such as Ti plasmid, and giving external stimulation to the promoter to regulate the expression of the above-described genes. The examples of the promoters usable herein are as follows:
   * A 35S promoter (possible to express constitutively)
   * A heat shock promoter (possible to express temperature dependently)
   * A Dex-inducible promoter (possible to express by the exposure to Dexamethason)]
   * A CHS-A promoter of petunia (petal specific expression in plants with colored petals and sugar-dependent expression not specific to petal but to leaf bud in *Arabitopsis*).
   In addition to these promoters, other conventional promoters in plant field are usable.
(2) A method to suppress the function of ROT3 or CYP90D 1:
   It is possible to suppress functions of a specific gene by an antisence RNA method (a method for transducing an altered gene so as to be transcribed in reverse direction) or by a RNAi method (a method for transducing an altered gene so as to be ligated a part of a gene tamdemly in forward and reverse and as to be transcribed throughout). The above-described method may be applied for suppressing genetic expression. Since the target genes are CYP90D1 (SEQ ID: 1) and ROT3 (=CYP90C1, #51 to #1625 of SEQ ID NO: 3) are revealed, targeted suppression of their expression is possible.
(3) A method of combination:
   It is prerequisite to prepare singly altered strains of CYP90D1 and ROT3 (=CYP90C1, #51 to #1625 of SEQ ID NO: 3), independently, then, two methods are possible: either crossing between them by classical genetics or direct transduction of both altered genes should bring about doubly altered strains.
(4) A method of precursor fermentation:
   There are successful examples for at least a part of genes responsible to brassinosteroid biosynthesis showing enzymatic activity when these genes are expressed in yeast cells. Using the methods of these examples and providing appropriate precursors, it is possible to artificially synthesize castasterone or brassinolide (the final and active product) in yeast cells or in eucaryotic cells, wherein combination of ROT3 and CYO90D1 or one of the above-described genes are expressed.

### Brief description of drawings:

Figure 1 shows the whole pathway of brassinosteroid biosynthesis.

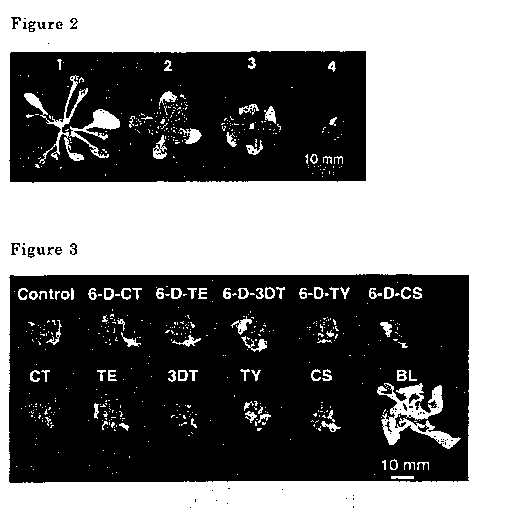

Figure 2 shows morphology of leaves of wild type (Ws-2)(No. 1), a strain of reference example 1 (suppression of ROT3 function) (No.2), a strain of reference example 2 (suppression of both ROT3 and CYP90D1 function)(No. 3 and 4) of *Arabidopsis* cultivated in the same condition. No.3 and No.4 show a partly effective and a strongly effective strain, respectively.
Figure 3 shows morphology of leaves of strains without function of ROT3 and CYP90D1 after the treatment with intermediates of brassinosteroid synthesis and brassinolide. Control: no treatment, 6-D-CT: treated with (hereinafter the same) 6-Deoxocathasterone, 6-D-TE:6-Deoxoteasterone,6-D-3DT: 3-Dehydro-6-deoxoteasterone, 6-D-TY: 6-Deoxotyphasterol , 6-D-CS: 6-Deoxocastasterone, CT: Cathasterone, TE: Teasterone, 3DT: 3-Dehydroteasterone, TY: Typhasterol, CS: Castasterone, BL :Brassinolide.

### Effects of the invention:

There are several practical defects in previous invention on the regulation of steps in biosynthetic pathway of steroid compounds, which show distinctive physiological activity in plants.

Namely, the regulatory factor of brassinosteroid biosynthesis previously elucidated is involved in the early steps of the biosynthetic pathway and enforced expression of the factor in transgenic plants brings about spindly growth of the whole plant and enlarges the plant. Therefore, there are no practical utility values for the above-described regulatory factor except for their occasional application. On the other hand, stopping a biosynthetic pathway in a transgenic plant resulted in miniaturization of the whole plant and, again, there are no practical utility except for their occasional application. In other words, conventional methods change the whole shape of a plant, which is practically not valuable. Practically usable and valuable transgenic plants are shown by the following examples: in horticulture, only the size of floral organs is large or only the size of leaves is small, or in improvement of vegetables, only the size of leaves is large. Therefore, conventional methods have difficulty in applying to biodesign of plants without the combination with a special expression-regulatory system. According to the present invention, in contrast to them, it becomes possible to control the size of a specific organ in a specific direction (specially in longitudinal direction) and the whole size of a plant by using the combination of ROT3 (=CYP90C1) with CYP90D1.

Furthermore, the present invention elucidated that ROT3 (=CYP90C1) and CYP90D1 cooperatively regulate the final step of brassinosteroid biosynthesis. Therefore, the invention could be used for various industrial applications using as chemical synthesis of brassinosteriod.

The following examples illustrate this invention, however, it is not intended to limit the scope of the invention.

### Manufacturing example 1.

As the strain knocked down the function of ROT3, the inventors used rot3-1 null mutant of *Arabidopsis* (Tsuge et al., Development 122: 1589-1600 (1996), a functional defect mutant of ROT3. The mutant cell line was seeded under sterilized conditions and cultured at 23°C under continuous illumination.

### Manufacturing example 2.

To get the strain knocked down the function of both ROT3 and CYP90D1, the inventors, first of all, isolated cDNA of CYP90D 1 from *Arabidopsis* using a primer set, ROT3h-cDNA-for: 5'-GTTAAAACACTAATGGACAC-3'(SEQ ID NO: 5); ROT3h-cDNA-rev: 5'-TGATTTATATTCTTTTGATCC-3'(SEQ ID NO: 6), which could specifically amplify the ROT3 homologue (CYP90D1). Then, the above-described CYP90D1 (SEQ ID NO: 1) clone was inserted to be transcribed in reverse direction from cauliflower mosaic virus 35S promoter into multipurpose vector pBI121, wherein hygromycin resistant gene was inserted as a selection marker and GUS protein coding region was removed. The construct was transduced into *Agrobacterium* (C58C1 Rif-resistant) and was introduced into *Arabidopsis* rot3-1 by in planta method, using conventional way of suspension culture medium of *Agrobacterium.* After the transformants were selected by hygromycin, transformants with homozygous inserted genes were isolated by self-pollination. Then, the strain was seeded under sterilized conditions and was cultured at 23°C under continuous illumination.

### Example 1

Figure 2 shows morphology of leaves of wild species (Ws-2)(No. 1), a strain of reference example 1 (suppression of RO3 function) (No.2), a strain of reference example 2 (suppression of both Rot3 and CYP90D1 function)(No. 3 and 4) of *Arabidopsis* cultivated in the same condition. While the leaves of the strain with suppressed function of ROT3 (Fig.2-2) are shorter in longitudinal direction compared to those of wild type (Fig.2-1), the leaves of the strain with suppressed function of both ROT3 and CYP90D1 (Fig2-3 & 4) are shorter further more than those of the above strains. In short, ROT3 and CYP90D1 are genes, which are cooperatively involved in biosynthetic pathway of brassinosteroid.

### Example 2

The strain with suppressed function of both ROT3 and CYP90D1 prepared in reference example 2 was cultured from seeds in sterilized conditions. The seeds of the above-described strain were seeded on the MS medium (solidified by 0.2% Gelrite) supplemented with 2% (w/v) sucrose and were conventionally cultured at 23°C under continuous illumination after seeding under sterilized conditions. On the other hand, aqueous solution (0.1 µM) of intermediates of brassinosteroid biosynthesis (e.g., 6-D-CT: 6-Deoxocathasterone, 6-D-TE: 6-Deoxoteasterone, 6-D-3DT: 3-Dehydro-6-deoxoteasterone6-D-TY: 6-Deoxotyphasterol 6-D-CS: 6-Deoxocastasterone, CT: Cathasterone, TE:Teasterone, 3DT: 3-Dehydroteasterone, TY:Typhasterol, CS:Castasterone) and brassinolide (BL) (BL was obtained from WAKO pharmaceutical Co. (made by FujiKagaku Industry, Co.) and other brassinosteroids were gifts from Dr. Shouzou Jujioka, RIKEN and Dr. Tohide Takatsu, Jouetsu University of Education) was prepared.
The above-described plants (strains of suppressed function of both ROT3 and CYP90D1) were cultured in the above-described aqueous solution at the level of submersion under the solution with gently shaking. In the case of the treatment of leaves with the above-described intermediates or brassionolide, leafstalks were cut out by a surgical knife after taking out the plants under sterilized conditions and were treated in the same way. Figure 3 shows the photographs of these leaves.
As shown in Fig.3, each brassinosteroid intermediate was not effective to plants without the function of ROT3 and CYP90D1, however, brassinolide (BL), the final product, induced large size of leaves and showed distinguished effects. Namely, ROT3 and CYP90D1 are cooperatively involved in the synthesis of brassinolide, the final product of the biosynthetic pathway of brassinosteriods.

### Example 3.

The concentrations of brassinosteroids were determined in wild strains (Ws-2), the strain of reference example 1 (rot3-1 and rot3-5, suppressed function of ROT3) and the strain of reference example 3 (rot3/CYP90D1, suppressed function of ROT3 and CYP90D1) of *Arabidoposis.* The concentration was determined by harvesting the ground part of the plants at the time of rosette formation by reaping, by freezing and drying, and by detecting using HPLC and GC-MS. The results are shown in Table 1.

**Table 1**

| | ng/g | | | |
|---|---|---|---|---|
| | Ws-2 | rot3-1 | rot3-5 | rot3/CYP90D1 |
| 6-Deoxoteasterone | 0.05 | 0.19 | 0.11 | 0.26 |
| 3-Deoxotyphasterol | 2.30 | 3.49 | 4.30 | 0.38 |
| 6-Deoxocastasterone | 2.60 | 1.88 | 4.00 | 0.034 |
| Teasterone | - | 0.004 | 0.02 | - |
| Typhasterol | 0.27 | 0.38 | 0.46 | 0.014 |
| Castasterone | 0.28 | 0.31 | 0.50 | 0.020 |
| Brassinolide | 0.20 | 0.04 | 0.06 | - |

| | | | | |
|---|---|---|---|---|
| Note: (-) in the table shows that the value is less than the limit of detection (0.001 ng/g). | | | | |

As shown in the table, in the strain (rot3-1 and rot3-5) with suppressed ROT3, the production of brassinolide decreased remarkably, as a consequence of this, the production of brassinosteroids in the previous stage (especially castasterone) increased. However, suppression of ROT3 did not completely block the production of brassinolide. In other words, ROT3 itself dose not completely regulate the biosynthesis of brassinolide.

On the other hand, in the strain (rot3/CYP90D1) with suppressed function of both ROT3 and CYP90D1, the amount of brassinolide extremely decreased among intermediates of brassionosteroid biosynthesis, which indicates that the pathway of the synthesis of brassinolide from castasterone is completely blocked by simultaneous suppression of both ROT3 and CYP90D1. In other words, the production of brassinolide is completely regulated by the combined action of ROT3 and CYP90D1.

## Claims

1. A polynucleotide having the nucleotide sequence of (1) or (2), and that of (3) or (4):
(1) Nucleotide sequence of SEQ ID NO: 1.
(2) Nucleotide sequences encoding either of the following proteins,
(a) A protein having the amino acid sequence of SEQ ID NO: 2.
(b) A protein having the amino acid of SEQ ID NO: 2, wherein one amino acid is deleted, substituted or added, and its expression stimulates brassinosteriod biosynthesis
(3) Nucleotide sequence of #51 to #1625 of SEQ ID NO: 3.
(4) Nucleotide sequence encoding either of the following proteins,
(c) A protein having the amino acid sequence of SEQ ID NO: 4.
(d) A protein having the amino acid sequence of SEQ ID NO: 4, wherein one amino acid is deleted, substituted or added, and its expression stimulates brassinosteriod biosynthesis.

2. A polynucleotide comprising a promoter and the polynucleotide of claim 1 wherein both of said nucleotide sequences are linked to said promoter in forward direction.

3. A polynucleotide comprising a promoter and the polynucleotide of claim 1, wherein at least one of said nucleotide sequence is linked to said promoter in reverse direction.

4. A plasmid comprising the polynucleotide according to any preceding claim.

5. A plant transformed by the polynucleotide according to any one of claims 1 to 3.

6. A method for changing the morphology of a plant, comprising the steps of transforming a plant by the polynucleotide of claim 1, and promoting or suppressing the expression of said polynucleotide.

7. A method for changing the morphology of a plant, wherein said plant is transformed by the polynucleotide according to either claim 2 or 3, said method comprising stimulating the promoter of said polynucleotide.

8. The plant with a morphology altered by the method of either claim 6 or 7.

9. A mixture or a complex of a protein of the following (a) or (b) and a protein of the following (c) or (d):
(a) A protein having the amino acid of SEQ ID NO: 2.
(b) A protein having the amino acid sequence of SEQ ID NO: 2, wherein one amino acid is deleted, substituted or added and its expression stimulates brassinosteriod biosynthesis.
(c) A protein having the amino acid of SEQ ID NO: 4.
(d) A protein having the amino acid sequence of SEQ ID NO: 4, wherein one amino acid is deleted, substituted or added, and its expression stimulates the biosynthesis of brassinosteriod.

## Patentansprüche

1. Polynucleotid, das die Nucleotidsequenz von (1) oder (2) und diejenige von (3) oder (4) aufweist:
(1) Nucleotidsequenz von SEQ ID NO: 1.
(2) Nucleotidsequenzen, die eines der folgenden Proteine codieren,
(a) ein Protein, das die Aminosäuresequenz von SEQ ID NO: 2 aufweist;
(b) ein Protein, das die Aminosäuresequenz von SEQ ID NO: 2 aufweist, in der eine Aminosäure deletiert, substituiert oder angefügt ist, und dessen Expression die Brassinosteroid-Biosynthese stimuliert.
(3) Nucleotidsequenz von Nr. 51 bis Nr. 1625 von SEQ ID NO: 3.
(4) Nucleotidsequenz, die eines der folgenden Proteine codiert,
(c) ein Protein, das die Aminosäuresequenz von SEQ ID NO: 4 aufweist;
(d) ein Protein, das die Aminosäuresequenz von SEQ ID NO: 4 aufweist, in der eine Aminosäure deletiert, substituiert oder angefügt ist, und dessen Expression die Brassinosteroid-Biosynthese stimuliert.

2. Polynucleotid, umfassend einen Promotor und das Polynucleotid nach Anspruch 1, in dem die beiden Nucleotidsequenzen an den Promotor in Vorwärtsrichtung gebunden sind.

3. Polynucleotid, umfassend einen Promotor und das Polynucleotid nach Anspruch 1, in dem mindestens eine der Nucleotidsequenzen an den Promotor in umgekehrter Richtung gebunden ist.

4. Plasmid, umfassend das Polynucleotid nach einem der vorangehenden Ansprüche.

5. Pflanze, die durch das Polynucleotid nach einem der Ansprüche 1 bis 3 transformiert ist.

6. Verfahren zum Verändern der Morphologie einer Pflanze, umfassend die Schritte des Transformierens einer Pflanze durch das Polynucleotid nach Anspruch 1 und des Förderns oder Unterdrückens der Expression des Polynucleotids.

7. Verfahren zum Verändern der Morphologie einer Pflanze, bei dem die Pflanze durch das Polynucleotid nach Anspruch 2 oder 3 transformiert wird, wobei das Verfahren das Stimulieren des Promotors des Polynucleotids umfasst.

8. Pflanze mit einer durch das Verfahren nach Anspruch 6 oder 7 veränderten Morphologie.

9. Gemisch oder Komplex aus einem Protein des folgenden (a) oder (b) und einem Protein des folgenden (c) oder (d):
(a) ein Protein, das die Aminosäuresequenz von SEQ ID NO: 2 aufweist;
(b) ein Protein, das die Aminosäuresequenz von SEQ ID NO: 2 aufweist, in der eine Aminosäure deletiert, substituiert oder angefügt ist, und dessen Expression die Brassinosteroid-Biosynthese stimuliert;
(c) ein Protein, das die Aminosäuresequenz von SEQ ID NO: 4 aufweist;
(d) ein Protein, das die Aminosäuresequenz von SEQ ID NO: 4 aufweist, in der eine Aminosäure deletiert, substituiert oder angefügt ist, und dessen Expression die Biosynthese von Brassinosteroid stimuliert.

## Revendications

1. Polynucléotide ayant la séquence nucléotidique de (1) ou (2), et celle de (3) ou (4) :
(1) séquence nucléotidique de la SEQ ID N° : 1,
(2) séquences nucléotidiques codant pour l'une ou l'autre des protéines suivantes,
(a) une protéine ayant la séquence d'acides aminés de la SEQ ID N° : 2,
(b) une protéine ayant la séquence d'acides aminés de la SEQ ID N° : 2, dans laquelle un acide aminé est délété, substitué ou ajouté, et son expression stimule la biosynthèse de brassinostéroïde,
(3) la séquence nucléotidique du N° 51 au N° 1625 de la SEQ ID N° : 3,
(4) une séquence nucléotidique codant pour l'une ou l'autre des protéines suivantes,
(c) une protéine ayant la séquence d'acides aminés de la SEQ ID N° : 4,
(d) une protéine ayant la séquence d'acides aminés de la SEQ ID N° : 4, dans laquelle un acide aminé est délété, substitué ou ajouté, et son expression stimule la biosynthèse de brassinostéroïde.

2. Polynucléotide comportant un promoteur et le polynucléotide de la revendication 1, dans lequel les deux desdites séquences nucléotidiques sont liées audit promoteur dans une direction directe.

3. Polynucléotide comportant un promoteur et le polynucléotide de la revendication 1, dans lequel au moins l'une desdites séquences nucléotidiques est liée audit promoteur dans une direction inverse.

4. Plasmide comportant le polynucléotide selon l'une quelconque des revendications précédentes.

5. Végétal transformé par le polynucléotide selon l'une quelconque des revendications 1 à 3.

6. Procédé pour changer la morphologie d'un végétal, comportant les étapes consistant à transformer un végétal par le polynucléotide de la revendication 1, et promouvoir ou supprimer l'expression dudit polynucléotide.

7. Procédé pour changer la morphologie d'un végétal, dans lequel ledit végétal est transformé par le polynucléotide de la revendication 2 ou 3, ledit procédé comportant la stimulation du promoteur dudit polynucléotide.

8. Végétal ayant une morphologie modifiée par le procédé selon la revendication 6 ou 7.

9. Mélange ou complexe d'une protéine ayant la séquence du point (a) ou (b) suivant et d'une protéine du point (c) ou (d) suivant :
(a) une protéine ayant la séquence d'acides aminés de la SEQ ID N° : 2,
(b) une protéine ayant la séquence d'acides aminés de la SEQ ID N° : 2, dans laquelle un acide aminé est délété, substitué ou ajouté, et son expression stimule la biosynthèse de brassinostéroïde,
(c) une protéine ayant la séquence d'acides aminés de la SEQ ID N° : 4,
(d) une protéine ayant la séquence d'acides aminés de la SEQ ID N° : 4, dans laquelle un acide aminé est délété, substitué ou ajouté, et son expression stimule la biosynthèse de brassinostéroïde.


### SEQUENCE LISTING

<110> Japan Science and Technology Corporation
<120> Gene participating in the synthesis of brassinosteroid
<130> FS03-311PCT
<160> 6
<210> 1
<211> 1473
<212> DNA
<213> Arabidopsis thaliana
<400> 1
   atggacactt cttcttcact tttgttcttc tccttcttct tctttatcat catcgtcatc     60
   ttcaacaaga tcaacggtct cagatcatcc ccagcttcaa agaaaaaact taatgatcat    120
   catgttacat cccagagtca cggaccaaag tttccacacg gaagcttggg atggcccgtc    180
   atcggtgaaa ccatcgagtt cgtctcttct gcttactcag accgtcctga gagtttcatg    240
   gacaagcgtc gtctcatgta tgggagagtg tttaagtcgc atatttttgg aacggcgacg    300
   atcgtgtcga cggatgctga agtgaacaga gccgttttac agagcgactc gacagctttc    360
   gtgccgtttt acccaaaaac ggtaagggag ctaatgggaa aatcgtcgat acttcttatc    420
   aacgggagtt tacatagacg gttccatgga ttagtcggtt ctttcttaaa gtcgccactt    480
   ctcaaagctc aaatcgttag agacatgcac aagtttttgt cggaatccat ggatctatgg     540
   tccgaggacc aacctgtgct cctccaagac gtctccaaga ctgttgcatt caaagtactt    600
   gccaaggcat tgataagtgt agagaaagga gaagatttag aagagctaaa gagagagttt    660
   gaaaatttca tatcaggact catgtcatta ccaattaact tccctggaac gcaactccat    720
   agatctctcc aagctaagaa gaatatggtg aagcaagttg aaagaatcat agaaggcaaa    780
   attaggaaaa caaagaacaa ggaggaagat gatgttattg caaaggatgt tgtggatgtg    840
   ttgcttaagg actcaagtga acatttaact cacaatttga ttgctaacaa tatgatcgac    900
   atgatgatcc ctggccacga ttctgtccct gtcctcatta cccttgccgt caaattcctc    960
   tctgattctc ctgctgccct caatctccta acgaaaaaca tgaagctgaa aagtttgaag   1020
   gaattgacag gagagccact atattggaat gactacttgt cgttaccttt aacacaaaag   1080
   gtgattacag agacactgag aatgggaaat gttataattg gagtgatgag aaaggcgatg   1140
   aaagatgttg aaataaaagg atatgtgata ccaaaaggat ggtgtttctt ggcctatctc   1200
   agatcagttc atcttgatga agcttattat gagtctccgt acaaatttaa tccctggaga   1260
   tggcaagaaa gggacatgaa cacgagtagt ttcagtcctt ttggaggtgg tcagagattg   1320
   tgccctggtc tcgatttggc tcgtcttgaa acttcagttt ttcttcacca tcttgtcact   1380
   cgcttcagat ggatagcaga agaagacaca atcataaact tcccaacggt gcatatgaag   1440
   aacaaattac ccatttggat caaaagaata taa                                1473
<210> 2
<211> 490
<212> PRT
<213> Arabidopsis thaliana
<400> 2
   Met Asp Thr Ser Ser Ser Leu Leu Phe Phe Ser Phe Phe Phe Phe Ile
   1 5 10 15
   Ile Ile Val Ile Phe Asn Lys Ile Asn Gly Leu Arg Ser Ser Pro Ala
   20 25 30
   Ser Lys Lys Lys Leu Asn Asp His His Val Thr Ser Gln Ser His Gly
   35 40 45
   Pro Lys Phe Pro His Gly Ser Leu Gly Trp Pro Val Ile Gly Glu Thr
   50 55 60
   Ile Glu Phe Val Ser Ser Ala Tyr Ser Asp Arg Pro Glu Ser Phe Met
   65 70 75 80
   Asp Lys Arg Arg Leu Met Tyr Gly Arg Val Phe Lys Ser His Ile Phe
   85 90 95
   Gly Thr Ala Thr Ile Val Ser Thr Asp Ala Glu Val Asn Arg Ala Val
   100 105 110
   Leu Gln Ser Asp Ser Thr Ala Phe Val Pro Phe Tyr Pro Lys Thr Val
   115 120 125
   Arg Glu Leu Met Gly Lys Ser Ser Ile Leu Leu Ile Asn Gly Ser Leu
   130 135 140
   His Arg Arg Phe His Gly Leu Val Gly Ser Phe Leu Lys Ser Pro Leu
   145 150 155 160
   Leu Lys Ala Gln Ile Val Arg Asp Met His Lys Phe Leu Ser Glu Ser
   165 170 175
   Met Asp Leu Trp Ser Glu Asp Gln Pro Val Leu Leu Gln Asp Val Ser
   180 185 190
   Lys Thr Val Ala Phe Lys Val Leu Ala Lys Ala Leu Ile Ser Val Glu
   195 200 205
   Lys Gly Glu Asp Leu Glu Glu Leu Lys Arg Glu Phe Glu Asn Phe Ile
   210 215 220
   Ser Gly Leu Met Ser Leu Pro Ile Asn Phe Pro Gly Thr Gln Leu His
   225 230 235 240
   Arg Ser Leu Gln Ala Lys Lys Asn Met Val Lys Gln Val Glu Arg Ile
   245 250 255
   Ile Glu Gly Lys Ile Arg Lys Thr Lys Asn Lys Glu Glu Asp Asp Val
   260 265 270
   Ile Ala Lys Asp Val Val Asp Val Leu Leu Lys Asp Ser Ser Glu His
   275 280 285
   Leu Thr His Asn Leu Ile Ala Asn Asn Met Ile Asp Met Met Ile Pro
   290 295 300
   Gly His Asp Ser Val Pro Val Leu Ile Thr Leu Ala Val Lys Phe Leu
   305 310 315 320
   Ser Asp Ser Pro Ala Ala Leu Asn Leu Leu Thr Lys Asn Met Lys Leu
   325 330 335
   Lys Ser Leu Lys Glu Leu Thr Gly Glu Pro Leu Tyr Trp Asn Asp Tyr
   340 345 350
   Leu Ser Leu Pro Leu Thr Gln Lys Val Ile Thr Glu Thr Leu Arg Met
   355 360 365
   Gly Asn Val Ile Ile Gly Val Met Arg Lys Ala Met Lys Asp Val Glu
   370 375 380
   Ile Lys Gly Tyr Val Ile Pro Lys Gly Trp Cys Phe Leu Ala Tyr Leu
   385 390 395 400
   Arg Ser Val His Leu Asp Glu Ala Tyr Tyr Glu Ser Pro Tyr Lys Phe
   405 410 415
   Asn Pro Trp Arg Trp Gln Glu Arg Asp Met Asn Thr Ser Ser Phe Ser
   420 425 430
   Pro Phe Gly Gly Gly Gln Arg Leu Cys Pro Gly Leu Asp Leu Ala Arg
   435 440 445
   Leu Glu Thr Ser Val Phe Leu His His Leu Val Thr Arg Phe Arg Trp
   450 455 460
   Ile Ala Glu Glu Asp Thr Ile Ile Asn Phe Pro Thr Val His Met Lys
   465 470 475 480
   Asn Lys Leu Pro Ile Trp Ile Lys Arg Ile
   485 490
<210> 3
<211> 1934
<212> DNA
<213> Arabidopsis thaliana
<400> 3
   tgtgcttagg catatagtta ttcccaagaa accggtttaa ctgtttacgt atgcaacctc 60
   cggcaagcgc aggacttttc cggtcgccgg aaaatctccc ttggccttat aattacatgg 120
   attatttggt cgctggtttc ttggttttga cggccggaat acttctccgt ccatggctct 180
   ggtttcgtct acgaaactcg aaaacgaaag atggagatga agaagaagat aatgaggaga 240
   agaagaaggg aatgattcca aacggaagct taggctggcc ggtgatcgga gaaaccctaa 300
   acttcatcgc ttgtggttat tcttctcggc ctgttacctt catggacaaa cgaaagtctt 360
   tatacgggaa agtgttcaaa acgaacataa tagggacacc aatcataata tcaaccgatg 420
   cagaggtgaa taaagtggtg ctccaaaacc atgggaacac atttgtccct gcatacccta 480
   aatcaattac ggaactactt ggagaaaact ctattctcag catcaatgga cctcatcaaa 540
   aaaggcttca cacgctcatt ggcgcgttcc tcagatctcc tcacctcaaa gaccggatca 600
   ctcgagacat tgaggcctcg gttgttctca ctttggcgtc ttgggctcaa cttccattgg 660
   ttcatgttca ggatgagatc aaaaagatga cgtttgagat attagtaaaa gtgttgatga 720
   gcacatctcc tggtgaagat atgaacattc tcaaacttga gttcgaagaa ttcatcaaag 780
   gtttgatttg tatcccaatc aaattccctg gcactagact ctacaaatcc ttaaaggcga 840
   aagagaggtt aataaagatg gtaaaaaagg ttgtggagga gagacaagtg gcgatgacaa 900
   cgacgtctcc ggcaaatgac gtggtggacg tacttctaag agacggtggt gattcagaga 960
   agcaatctca accgtcagat ttcgtcagcg gaaagatcgt agagatgatg atacccggag 1020
   aggaaacaat gccaacggcg atgaccttgg ctgtcaaatt cttaagtgac aaccccgtcg 1080
   ctctagccaa actcgtggag gagaatatgg agatgaagag gcgtaaattg gaattgggag 1140
   aagaatacaa gtggaccgat tatatgtctc tctcttttac tcaaaatgtg ataaacgaaa 1200
   cgcttagaat ggctaacatt attaacgggg tgtggaggaa agctctcaag gatgtagaaa 1260
   ttaaaggtta cttaataccg aaaggatggt gtgtattggc atcattcata tcggttcaca 1320
   tggatgaaga catttatgat aatccctatc aattcgatcc gtggagatgg gacagaatta 1380
   atggatcggc aaacagcagt atttgcttca caccctttgg tggtgggcaa aggctatgtc 1440
   ctggtttaga gctgtcgaag ctcgaaatat ccatctttct tcaccacctt gtaacccggt 1500
   acagttggac ggctgaggaa gacgagatag tgtcatttcc gactgtgaag atgaagcgga 1560
   ggctcccgat ccgagtggct actgtagatg atagtgcttc tccgatctca cttgaagatc 1620
   attaatagat catttcaaag aacaaaactg tttgtgcaaa gaggaagcag agaagtaaac 1680
   aaatgatctt attaacaaat agtagagaag agaagcaaac aagattggtg ggtaagacag 1740
   aaagaacnaa acgtacagct agtgatggct caaagatgag agattctaat tataattttt 1800
   tttgtttgtc atgtcaaatt ataagcgttg gttaggttgt ccctttctct tttatttatc 1860
   gtaccaaacg caagttgaga tatgattcca tatatatgga tgatagatat gtatattaat 1920
   atatagcggc cggg 1934
<210> 4
<211> 524
<212> PRT
<213> Arabidopsis thaliana
<400> 4
   Met Gln Pro Pro Ala Ser Ala Gly Leu Phe Arg Ser Pro Glu Asn Leu
   1 5 10 15
   Pro Trp Pro Tyr Asn Tyr Met Asp Tyr Leu Val Ala Gly Phe Leu Val
   20 25 30
   Leu Thr Ala Gly Ile Leu Leu Arg Pro Trp Leu Trp Phe Arg Leu Arg
   35 40 45
   Asn Ser Lys Thr Lys Asp Gly Asp Glu Glu Glu Asp Asn Glu Glu Lys
   50 55 60
   Lys Lys Gly Met Ile Pro Asn Gly Ser Leu Gly Trp Pro Val Ile Gly
   65 70 75 80
   Glu Thr Leu Asn Phe Ile Ala Cys Gly Tyr Ser Ser Arg Pro Val Thr
   85 90 95
   Phe Met Asp Lys Arg Lys Ser Leu Tyr Gly Lys Val Phe Lys Thr Asn
   100 105 110
   Ile Ile Gly Thr Pro Ile Ile Ile Ser Thr Asp Ala Glu Val Asn Lys
   115 120 125
   Val Val Leu Gln Asn His Gly Asn Thr Phe Val Pro Ala Tyr Pro Lys
   130 135 140
   Ser Ile Thr Glu Leu Leu Gly Glu Asn Ser Ile Leu Ser Ile Asn Gly
   145 150 155 160
   Pro His Gln Lys Arg Leu His Thr Leu Ile Gly Ala Phe Leu Arg Ser
   165 170 175
   Pro His Leu Lys Asp Arg Ile Thr Arg Asp Ile Glu Ala Ser Val Val
   180 185 190
   Leu Thr Leu Ala Ser Trp Ala Gln Leu Pro Leu Val His Val Gln Asp
   195 200 205
   Glu Ile Lys Lys Met Thr Phe Glu Ile Leu Val Lys Val Leu Met Ser
   210 215 220
   Thr Ser Pro Gly Glu Asp Met Asn Ile Leu Lys Leu Glu Phe Glu Glu
   225 230 235 240
   Phe Ile Lys Gly Leu Ile Cys Ile Pro Ile Lys Phe Pro Gly Thr Arg
   245 250 255
   Leu Tyr Lys Ser Leu Lys Ala Lys Glu Arg Leu Ile Lys Met Val Lys
   260 265 270
   Lys Val Val Glu Glu Arg Gln Val Ala Met Thr Thr Thr Ser Pro Ala
   275 280 285
   Asn Asp Val Val Asp Val Leu Leu Arg Asp Gly Gly Asp Ser Glu Lys
   290 295 300
   Gln Ser Gln Pro Ser Asp Phe Val Ser Gly Lys Ile Val Glu Met Met
   305 310 315 320
   Ile Pro Gly Glu Glu Thr Met Pro Thr Ala Met Thr Leu Ala Val Lys
   325 330 335
   Phe Leu Ser Asp Asn Pro Val Ala Leu Ala Lys Leu Val Glu Glu Asn
   340 345 350
   Met Glu Met Lys Arg Arg Lys Leu Glu Leu Gly Glu Glu Tyr Lys Trp
   355 360 365
   Thr Asp Tyr Met Ser Leu Ser Phe Thr Gln Asn Val Ile Asn Glu Thr
   370 375 380
   Leu Arg Met Ala Asn Ile Ile Asn Gly Val Trp Arg Lys Ala Leu Lys
   385 390 395 400
   Asp Val Glu Ile Lys Gly Tyr Leu Ile Pro Lys Gly Trp Cys Val Leu
   405 410 415
   Ala Ser Phe Ile Ser Val His Met Asp Glu Asp Ile Tyr Asp Asn Pro
   420 425 430
   Tyr Gln Phe Asp Pro Trp Arg Trp Asp Arg Ile Asn Gly Ser Ala Asn
   435 440 445
   Ser Ser Ile Cys Phe Thr Pro Phe Gly Gly Gly Gln Arg Leu Cys Pro
   450 455 460
   Gly Leu Glu Leu Ser Lys Leu Glu Ile Ser Ile Phe Leu His His Leu
   465 470 475 480
   Val Thr Arg Tyr Ser Trp Thr Ala Glu Glu Asp Glu Ile Val Ser Phe
   485 490 495
   Pro Thr Val Lys Met Lys Arg Arg Leu Pro Ile Arg Val Ala Thr Val
   500 505 510
   Asp Asp Ser Ala Ser Pro Ile Ser Leu Glu Asp His
   515 520
<210> 5
<211> 20
<212> DNA
<213> Artificial sequence
<400> 5
   gttaaaacac taatggacac 20
<210> 6
<211> 21
<212> DNA
<213> Artificial sequence
<400> 6
   tgatttatat tcttttgatc c 21